(19) Europäisches Patentamt
European Patent Office
Office européen des brevets

(11) Veröffentlichungsnummer: **0 088 327**
**B1**

(12) # EUROPÄISCHE PATENTSCHRIFT

(45) Veröffentlichungstag der Patentschrift: **16.07.86**

(51) Int. Cl.⁴: **B 01 J 27/18**, C 07 C 57/055

(21) Anmeldenummer: **83101938.5**

(22) Anmeldetag: **28.02.83**

(54) **Verfahren zur Herstellung von Methacrylsäure.**

(30) Priorität: **10.03.82 DE 3208572**

(43) Veröffentlichungstag der Anmeldung: **14.09.83 Patentblatt 83/37**

(45) Bekanntmachung des Hinweises auf die Patenterteilung: **16.07.86 Patentblatt 86/29**

(84) Benannte Vertragsstaaten: **BE DE FR GB IT NL**

(56) Entgegenhaltungen:
**EP - A - 0 088 328**
**DE - A - 2 429 095**
**DE - A - 2 523 757**
**DE - A - 2 832 132**
**DE - A - 2 952 455**
**DE - A - 3 010 434**
**GB - A - 1 438 806**
**US - A - 3 965 163**
**US - A - 4 272 408**

(73) Patentinhaber: **BASF Aktiengesellschaft, Carl-Bosch-Strasse 38, D-6700 Ludwigshafen (DE)**

(72) Erfinder: **Krabetz, Richard, Dr., Unterer Waldweg 8, D-6719 Kirchheim (DE)**
Erfinder: **Duembgen, Gerd, Dr., Sudetenstrasse 4, D-6701 Dannstadt-Schauernheim (DE)**
Erfinder: **Nees, Friedbert, Dr., Fichtestrasse 4, D-7513 Stutensee (DE)**
Erfinder: **Merger, Franz, Dr., Max-Slevogt-Strasse 25, D-6710 Frankenthal (DE)**
Erfinder: **Fouquet, Gerd, Dr., Maxburgstrasse 2, D-6730 Neustadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 088 327 B1

ACTORUM AG

**Beschreibung**

Es sind zahlreiche Oxidationskatalysatoren und deren Verwendung zur Herstellung von Methacrylsäure durch Gasphasenoxidation von Methacrolein vorgeschlagen worden. Diese befriedigen jedoch nicht oder nur zum Teil die Anforderungen des technischen Betriebes hinsichtlich hoher Selektivität bei hohen Methacroleinumsätzen und hohen Raumbelastungen über lange Betriebszeiten.

Aus der DE-A 2 952 455 sind z.B. Katalysatoren bekannt, die Mo, Cu, P, Sb und Cs und/oder Ca enthalten. Mit diesen Katalysatoren werden jedoch nur unbefriedigende Selektivitäten von 76% für die Methacrylsäurebildung bei Methacroleinumsätzen von nur 75% erreicht. In der DE-A 2 523 757 werden Katalysatoren vorgeschlagen, die zusätzlich zu Mo, Cu und P wenigstens ein Alkalimetall und wenigstens ein Metall, ausgewählt aus der Gruppe Sb, V, W, Fe, Mn und Sn enthalten. Mit diesen Katalysatoren werden zwar im Dauerbetrieb Umsätze an Methacrolein bis 91,5% und Selektivitäten von 82% erzielt, die niedrigen Durchsätze (space velocity) von 1000 $h^{-1}$ und relativ hohen Temperaturen von 325°C und mehr befriedigen jedoch nicht bei technischem Betrieb. Auch Oxidationskatalysatoren der aus der DE-A 3 010 434 bekannten Art, die Mo, P, V sowie gegebenenfalls As und Cu oder andere kationische Elemente enthalten, zeigen zwar hohe katalytische Wirksamkeit, jedoch nur bei technisch uninteressanten Katalysatorteilchengrössen unter 2 mm und verhältnismässig hohen Temperaturen von 330°C. Oxidationskatalysatoren, die in Gegenwart hoher Chlorionenkonzentrationen von etwa 1 bis 5 Äquivalenten je Äquivalent Molybdän hergestellt werden, wie beispielsweise die Mo, P und W enthaltenden Katalysatoren der bekanntgemachten europäischen Anmeldung EP-A 10 429 oder die Mo, P, Sb und gegebenenfalls W enthaltenden Katalysatoren gemäss der US-A 3 965 163 zeigen zwar eine verhältnismässig gute katalytische Wirksamkeit bei kurzen Betriebszeiten, doch ist es schwierig, langlebige und ausreichend selektive Katalysatoren dieser Art reproduzierbar herzustellen. Ausserdem neigen die genannten Katalysatoren zur verstärkten Bildung von Essigsäure, wenn sie in technisch sinnvollen Teilchengrössen von mindestens 3 mm und mehr eingesetzt werden. Aus der DE-A 2 832 132 sind weiter oxidische Katalysatoren bekannt, die Mo, P, As, Cu und Cr enthalten und die in Anwesenheit oder Abwesenheit einer dibasischen Carbonsäure, Oxycarbonsäure, Mannit oder Pyrogallol als Reduktionsmittel hergestellt sind. Die Eigenschaften dieser und der obengenannten Katalysatoren sind im allgemeinen jedoch dann unbefriedigend, wenn für die Herstellung von Methacrylsäure als Rohstoff Methacrolein eingesetzt wird, das durch Kondensation von Propanol mit Formaldehyd hergestellt ist. Dieses enthält als herstellungsbedingte Verunreinigungen neben nicht umgesetztem Propanol organische Amine, Dimere des Methacroleins und Methylpentenal. Bereits geringe Mengen dieser Verunreinigungen führen aber im allgemeinen zu einer Leistungsminderung derartiger Katalysatoren.

Es bestand daher ein technisches Interesse an einem Verfahren zur Oxidation von Methacrolein zu Methacrylsäure in der Gasphase, das beim Einsatz technischer Methacroleinqualitäten und technisch bei Festbettreaktionen üblicher Teilchengrössen des Katalysators hohe Ausbeuten und eine geringe Bildung an Nebenprodukten auch bei hohen Raumbelastungen über lange Betriebszeiten gewährleistet.

Es wurde nun gefunden, dass man Methacrylsäure durch Oxidation von Methacrolein mit Sauerstoff und Wasserdampf enthaltenden Gasgemischen bei Temperaturen von 200 bis 340°C mit Molybdän enthaltenden Katalysatoren und Abtrennen der Methacrylsäure aus den Reaktionsgasen in an sich üblicher Weise mit Vorteil herstellen kann, indem man die Oxidation an einem Katalysator der allgemeinen Formel

$$Mo_{12}P_aW_bSb_cAs_dCu_eX_fY_gO_x$$

durchführt, in der

| | |
|---|---|
| X | für ein oder mehrere Elemente aus der Gruppe Nb, Fe, Mn, Sn und/oder Cr, |
| Y | für ein Alkalimetall, |
| a | für 0,1 bis 3, |
| b | für 0,1 bis 4, |
| c | für > 0 bis 2, |
| d | für 0 bis 1, |
| d+e | für > 0 bis 2, |
| e | für 0 bis 1, |
| f | für 0 bis 1,5 |
| e+f | für 0 bis 2, |
| g | für 0 bis <1 und |
| x | für die zur Absättigung der Valenzen der anderen Bestandteile formal erforderliche Anzahl der Sauerstoffatome steht. |

Die gegebenenfalls vorhandenen $NH_4^+$-Ionen sind in die Formel aus formalen Gründen nicht mit aufgenommen. Die Katalysatoren können andere Komponenten in Mengen, die durch den natürlichen Gehalt der eingesetzten Rohstoffe gegeben sind, enthalten, beispielsweise Alkalimetalle wie Kalium und Natrium.

Die Konzentration der Alkalimetalle soll jedoch unter 0,1, vorzugsweise unter 0,035 Atomen je 12 Atome Molybdän liegen, da der Einfluss der Alkalimetalle eher schädlich ist.

Hinsichtlich der Zusammensetzung werden solche Oxidationskatalysatoren der oben genannten Formel bevorzugt, in denen

| | |
|---|---|
| a | für 0,5 bis 2, |
| b | für 0,5 bis 3, |
| c | für 0,2 bis 1,5, |
| d | für 0,01 bis 0,5, |
| e | für 0,01 bis 0,5 und |
| f | für 0 bis 1,0, insbesondere > 0 bis 1 stehen. |

Von den Komponenten der Gruppe X wird Nb und/oder Eisen, gegebenenfalls in Kombination mit einer weiteren Komponente dieser Gruppe, vorgezogen.

Die Katalysatoren und deren Herstellung sind Gegenstand der europäischen Patentanmeldung EP-A 88 328. Sie werden im allgemeinen so hergestellt, dass Verbindungen der Einzelkomponenten in wässrigem Medium, d.h. in wässriger Lösung oder Suspension, unter Bedingungen, die zur Bildung der phosphorhaltigen Heteropolysäuren des Molybdäns und Wolframs oder ihrer Salze führen, vereinigt, anschliessend getrocknet und verformt und schliesslich vorteilhaft bei höheren Temperaturen durch Calcinieren aktiviert werden.

Geeignet als Molybdän- und Wolframquelle sind beispielsweise Molybdänsäure, Ammoniummolybdat, Phosphomolybdänsäure und ihr Ammoniumsalz, Wolframsäure, Ammoniumwolframat, Phosphowolframsäure und ihr Ammoniumsalz. Obwohl auch andere Verbindungen eingesetzt werden können, werden die genannten Verbindungen bevorzugt, insbesondere der Einsatz der Phosphowolframsäure als Wolfram- und der Einsatz von Ammoniummolybdat, Molybdänsäure und Phosphomolybdänsäure als Molybdänquelle. Arsen wird vorteilhaft als Oxid bzw. Säure oder als Ammoniumsalz der Säuren eingesetzt. Als Phosphorquelle können neben den obengenannten Heteropolysäuren verschiedene Verbindungen eingesetzt werden, bevorzugt werden jedoch die Phosphorsäure und ihre Ammoniumsalze. Die kationischen Elemente können beispielsweise in Form der Oxide, Carbonate, Nitrate, Chloride, Fluoride, Formiate, Oxalate oder Acetate eingesetzt werden. Bevorzugt wird jedoch der Einsatz in Form der Salze niedermolekularer Mono- und Dicarbonsäuren. Die Anwesenheit reduzierend wirkender organischer Stoffe, insbesondere von niedermolekularen Monocarbonsäuren, wie Ameisensäure, Essigsäure, Di- und Oxycarbonsäuren, wie Oxalsäure, Weinsäure, Zitronensäure oder ihrer Salze, vorzugsweise Ameisensäure allein oder in Kombination mit den oben genannten Säuren, insbesondere Essigsäure, in der Ausgangslösung und während der Herstellung ist im allgemeinen vorteilhaft. Die Carbonsäuren können in Mengen von 0,02 bis 2 Mol, vorzugsweise 0,05 bis 1,5 Mol je Mol Molybdän zugesetzt werden. Höhere Chlorionenkonzentrationen können dagegen in manchen Fällen schädigend auf die Katalysatoren wirken. Die Chlorionenmenge in der Ausgangslösung soll daher vorzugsweise unter 0,3, insbesondere unter 0,25 Mol je Mol Molybdän liegen.

Die Vereinigung der Komponenten kann bei Raumtemperatur oder auch erhöhter Temperatur durchgeführt werden. Man kann beispielsweise die wässrigen Lösungen oder Suspensionen der Molybdänsäure, Phosphorsäure, Arsensäure, des Antimon(III)oxids und Kupferoxids oder Kupfersalzes mischen und anschliessend beispielsweise 2 bis 24 Stunden unter Rückfluss kochen. In einer anderen Ausführungsform werden die wässrigen Lösungen wasserlöslicher Verbindungen der Komponenten, beispielsweise Ammoniumheptamolybdat, Di-ammoniumphosphate, Di-ammoniumarsenat oder Arsensäure und Antimontrichlorid, in salz- oder vorzugsweise in ameisen-, wein-, bernstein- oder zitronensaurer Lösung bei Raumtemperatur gemischt, mit der wässrigen Lösung von z.B. der Phosphowolframsäure vereinigt und nach Zugabe der kationischen Zusatzkomponenten bei erhöhter Temperatur entwässert.

Die Entwässerung bzw. Trocknung der wässrigen Suspension der Komponenten geschieht im allgemeinen durch Eindampfen in Rührkesseln bei Temperaturen unter 140°C oder durch Sprühtrocknung bei Austrittstemperaturen zwischen 80 und 140°C.

Nach dem Trocknen werden die erhaltenen Massen meist auf Teilchengrössen von 200 bis 1200 µm gemahlen und gegebenenfalls nach Zusatz üblicher Träger, wie $SiO_2$ oder Aluminiumoxiden, sowie gegebenenfalls von Gleitmitteln, wie Graphit, zu Kugeln, Tabletten, Ringen oder anderen Formen verpresst. Man kann dann anschliessend in der Luft, unter Stickstoff oder schwach reduzierender Atmosphäre bei geringen Gasströmungen und Temperaturen von 180 bis 400°C, vorzugsweise von 220 bis 380°C, insbesondere von 320 bis 360°C calcinieren bzw. aktivieren. Die Trägerstoffe können auch während des Eindampfens der Katalysatorsuspension zugegeben werden, wodurch die Katalysatorkomponenten auf den Trägern abgeschieden werden. Schliesslich können auch die getrockneten und gemahlenen Katalysatormassen ohne Zusatz von Trägern bei den genannten Temperaturen calciniert und anschliessend zu geformten Gebilden verarbeitet oder auf Träger, insbesondere auf kugelförmige Träger, in Form von Schalen in an sich üblicher Weise, z.B. nach den aus den DE-A 2 909 670 und 2 909 671 bekannten Verfahren, aufgebracht werden. Die katalytisch aktiven Massen weisen nach der Calcinierung ausschliesslich die Struktur einer gestörten Heteropolysäure oder ihrer Salze mit charakteristischen Röntgenbeugungslinien auf. Sie sind besonders für die Oxidation von Methacrolein zu Methacrylsäure unter an sich üblichen Bedingungen in der Gasphase geeignet, insbesondere dann, wenn als Ausgangsmaterial Methacrolein verwendet wird, das durch Kondensation von Formaldehyd mit Propanal hergestellt ist.

Bei der erfindungsgemässen Gasphasenoxidation von Methacrolein werden als Oxidationsmittel Sauerstoff und Wasserdampf enthaltende Gasgemische eingesetzt, die über die im allgemeinen fest in Katalysatorbetten angeordnete Katalysatoren geleitet werden. Man arbeitet dabei im allgemeinen bei Drücken von 1 bis 5 bar, vorteilhaft von 1 bis 2,5 bar. Im allgemeinen beträgt bei dem Verfahren die auf Standardbedingungen bezogene Verweilzeit der Methacrolein enthaltenden Gasgemische 0,5 bis 5 sec. und Verweilzeiten von 1 bis 3 sec. bei Temperaturen im Bereich von 200 bis 340°C, insbesondere bei 220 bis 320°C, werden vorgezogen. Zusätzlich zu Sauerstoff, Methacrolein und Wasserdampf enthalten die Reaktionsgase im allgemeinen indifferente Gase, insbesondere Stickstoff, und der

Sauerstoff wird im allgemeinen als Luft zugeführt. Er kann jedoch auch als Reinsauerstoff eingesetzt werden. Zudem enthält das Reaktionsgas im allgemeinen Kohlenoxide, insbesondere dann, wenn nach der Abtrennung der entstandenen Methacrylsäure restliches Reaktionsabgas als Verdünnungsmittel zusammen mit nichtumgesetztem Methacrolein in die Oxidationsreaktion zurückgeführt wird. Im Reaktionsgas beträgt das molare Verhältnis von Methacrolein:Sauerstoff:Wasser:Inertgas meist 1:(1 bis 6):(1 bis 20):(4 bis 50), vorzugsweise 1:(1,5 bis 4):(2 bis 10):(6 bis 30). Die Abtrennung der Methacrylsäure von den heissen Reaktionsabgasen kann in an sich üblicher Weise, meist durch Abschrecken mit Wasser durchgeführt werden.

Das Methacrolein kann nach verschiedenen Verfahren gewonnen sein, z.B. durch Gasphasenoxidation von tert. Butylalkohol, Isobutylen oder $C_4$-Gemischen oder durch Kondensation von Propionaldehyd mit Formaldehyd. Das erfindungsgemässe Verfahren ist von besonderem Vorteil, wenn Methacrolein eingesetzt wird, das durch Kondensation von Propionaldehyd mit Formaldehyd in Gegenwart von Salzen sekundäre Amine oder mit Aminalen in Gegenwart von Säuren in wässriger Lösung hergestellt ist. Technische Qualitäten, die auf diese Weise hergestellt sind, haben im allgemeinen einen Reinheitsgrad von 94 bis 99% und enthalten neben nicht umgesetztem Propionaldehyd geringe Mengen organische Amine, wie Diethylamin oder Diethanolamin, Methylpentenal und Dimere des Methacroleins. Die Reinheitsangaben beziehen sich auf wasserfreies Rohmethacrolein, das bis zu 3,5 Gew.% Wasser enthalten kann. Soweit nichtumgesetztes Methacrolein und nicht kondensierte Reaktionsabgase in die Oxidationsreaktion zurückgeführt werden, kann das Synthesegasgemisch auch geringe Mengen leicht flüchtiger Nebenprodukte wie Kohlenoxide oder Acrolein enthalten.

Bei der technischen Durchführung des erfindungsgemässen Verfahrens arbeitet man meist in sogenannten Rohrbündelreaktoren in denen die Katalysatoren fest angeordnet sind. Zur Vermeidung von örtlichen Überhitzungen kann die Katalysatoraktivität derart verändert werden, dass sie im Reaktionsrohr in Strömungsrichtung der Reaktionsgase stetig oder in Stufen ansteigt. Dies kann z.B. durch Verdünnen des Katalysators mit weniger aktiven oder inaktiven Katalysator- oder Trägerformlingen oder durch Einsatz von 2 oder mehr Katalysatoren mit unterschiedlicher Aktivität und/oder Selektivität erfolgen. Es ist auch möglich, die erfindungsgemässe Oxidation des Methacroleins zu Methacrylsäure in der Wirbelschicht durchzuführen, fest angeordnete Katalysatorschichten werden jedoch vorgezogen.

Bei der Aufarbeitung der Reaktionsgase, bei der vor dem Waschen mit Wasser auch indirekt gekühlt werden kann, werden wässrige Lösungen der Methacrylsäure erhalten, die gegebenenfalls zusätzlich geringe Mengen Essigsäure, Maleinsäure und Acrylsäure enthalten. Aus den erhaltenen Lösungen der Methacrylsäure kann diese mit geeigneten Lösungsmitteln, wie beispielsweise Methylmethacrylat in an sich üblicher Weise extrahiert und entweder direkt mit Alkanolen verestert oder durch Destillation aus dem Extrakt abdestilliert und von den Nebenprodukten getrennt werden. Das nichtumgesetzte Methacrolein kann aus dem wässrigen Kondensat destilliert oder z.B. mit Wasserdampf ausgetrieben und der Oxidationsreaktion wieder zugeführt werden.

In den folgenden Beispielen wird Methacrolein eines Reinheitsgrades von 97 bis 99% eingesetzt, das neben Wasser und Propionaldehyd geringe Mengen an sekundären Aminen und Nebenprodukten der Methacroleinsynthese aus Propionaldehyd und Formaldehyd enthält. Die darin angegebenen Teile und Prozente beziehen sich, soweit nicht anders angegeben, auf das Gewicht. Die darin angegebenen Volumenteile verhalten sich zu den Gewichtsteilen wie das Liter zum Kilogramm.

Beispiel 1

Eine wässrige Lösung von 212 Teilen Ammoniumheptamolybdat in 600 Volumenteilen Wasser wurde nacheinander mit einer Lösung von 13,2 Teilen Di-ammoniumphosphat und 1,3 Teilen Diarsenpentoxid in 100 Vol.-Teilen Wasser, 22,6 Teilen Antimon(III)chlorid in einer Mischung von 6 Volumenteilen Ameisensäure und 20 Volumenteilen Wasser, einer Lösung von 19 Teilen Wolframatophosphorsäure in 50 Volumenteilen Wasser und schliesslich mit einer Lösung von 2,5 Teilen Kupfer(II)acetat in 100 Volumenteilen Wasser versetzt. Nach Eindampfen der Suspension auf dem Wasserbad bei etwa 85°C wurde Trockenmasse auf eine Körnung kleiner als 1,2 mm gemahlen und nach Zusatz von 2% Graphitpulver zu 3×3-mm-Tabletten verpresst. Die Formlinge wurden anschliessend 6 Stunden auf 355°C erhitzt. Der erhaltene Katalysator hatte die formale Zusammensetzung

$$Mo_{12}W_{0,9}P_{1,09}Sb_1As_{0,1}Cu_{0,12}O_x.$$

80 Volumenteile Katalysatortabletten wurden in einem salzbadbeheizten Reaktionsrohr von 16 mm Durchmesser eingefüllt. Über den Katalysator wurde mit einer Raumgeschwindigkeit von 1320 $h^{-1}$ eine Gasmischung aus 3,3 Vol.% Methacrolein, 9,1 Vol.% Sauerstoff, 29,5 Vol.% Wasserdampf und 58,1 Vol.% Stickstoff geleitet. Nach 7 Betriebstagen und bei einer Badtemperatur von 318°C betrug der Umsatz 94,3 Mol%, die Selektivität 85,8 Mol% und die Methacrylsäureausbeute 80,9 Mol%. Nach 10 Betriebstagen wurde ein Umsatz von 94,4 Mol%, eine Selektivität von 90,5 Mol% und eine Methacrylsäureausbeute von 85,4 Mol% gemessen. Die Nebenausbeute an Essigsäure betrug 2,6 Mol%. Nach 30 Betriebstagen betrug der Umsatz 94,1 Mol%, die Selektivität 86,2 Mol% und die Methacrylsäureausbeute 81,1 Mol%.

Vergleichsbeispiele

1A) Beispiel 1 wurde wiederholt mit der Änderung, dass der Zusatz des Antimonsalzes unterlassen wurde. Unter den Testbedingungen des Beispiels 1 wurde die optimale Ausbeute bei einer Badtemperatur von 280°C erreicht. Dabei wurde ein Umsatz von 75,5 Mol%, eine Selektivität von 70,8 Mol% und eine Methacrylsäureausbeute von 53,4 Mol% erreicht. Die Essigsäureausbeute betrug 4,6 Mol%.

1B) Beispiel 1 wurde mit der Änderung wiederholt, dass der Zusatz des Arsen- und Kupfersalzes unterlassen wurde. Unter den Testbedingungen von Beispiel 1 und bei der hinsichtlich der Methacrylsäure optimalen Badtemperatur von 300°C betrug der Umsatz 92,8 Mol%, die Selektivität 81 Mol%, die Methacrylsäureausbeute 75 Mol% und die Nebenausbeute an Essigsäure 4,6 Mol%.

1C) Die Herstellung des Katalysators gemäss Beispiel 1 wurde dahingehend geändert, dass der Zusatz der Kupfer- und Arsenverbindung unterlassen und die zugesetzte Ameisensäure durch konzentrierte Salzsäure ersetzt wurde. Unter den Testbedingungen von Beispiel 1 und bei einer Badtemperatur von 300°C betrug der Umsatz 82,6 Mol%, die Selektivität 77,4 Mol% und die Methacrylsäureausbeute 63,9 Mol%. Essigsäure wurde in einer Ausbeute von 6,5 Mol% gebildet.

Beispiel 2

In 2500 Teilen Wasser werden nacheinander 365 Teile Molybdatophosphorsäure und 48 Teile Wolframatophosphorsäure gelöst und dazu eine Lösung von 2,6 Teilen Arsenpentoxidhydrat in 100 Teilen Wasser gegeben. Man heizt das Gemisch auf 50°C und fügt 58,4 Teile Antimon(III)oxid und eine Lösung von 10 Teilen Kupfer(II)acetat in 200 Teilen Wasser zu. Man heizt das Gemisch auf 90°C und hält es 2 Stunden bei dieser Temperatur. Dann wird das Wasser auf dem Wasserbad abgedampft und 12 Stunden bei 90°C getrocknet. Der erhaltene Rückstand wird zerkleinert, mit 2% Graphit versetzt und zu 3×3-mm-Tabletten verpresst. Die Tabletten wurden 6 Stunden bei 350°C calciniert.

80 Volumenteile der calcinierten Katalysatortabletten wurden unter den in Beispiel 1 angegebenen Bedingungen bei einer Badtemperatur von 310°C getestet. Der Umsatz betrug 84 Mol%, die Selektivität 84,5 Mol% und die Methacrylsäureausbeute 71 Mol%.

Beispiele 3 bis 14

Entsprechend der Herstellungsvorschrift des Beispiels 1 wurden weitere Katalysatoren hergestellt, mit der Änderung, dass weitere Zusatzkomponenten eingeführt, oder der Zusatz von Arsen oder Kupfer unterlassen oder die Mengenverhältnisse der Komponenten geändert wurden. Die Katalysatoren wurden mit Ausnahme der Badtemperatur unter den Testbedingungen von Beispiel 1 geprüft. Die Katalysatorzusammensetzungen und Testbedingungen sind in Tabelle 1 zusammengefasst. Die Zusatzkomponenten wurden in folgender Form eingeführt: Mangan(II)acetat-4-hydrat, Eisen(II)oxalat, Niobpentoxid, Ammoniumchromat, Zinn(II)oxid, Kalium-, Rubidium-, Cäsiumnitrat.

Beispiele 15 bis 17

Entsprechend Beispiel 1 wurden weitere Katalysatoren hergestellt und getestet mit der Änderung, dass die zugesetzte Ameisensäure durch Weinsäure, Zitronensäure oder Oxalsäure ersetzt wurde. Die zugesetzten Säuremengen und die Testergebnisse sind in der Tabelle 2 zusammengefasst.

Tabelle 1

| Beispiel | | Badtemperatur °C | Umsatz Mol-% | Selektivität Mol-% | Ausbeute Mol-% | Nach Betriebstagen |
|---|---|---|---|---|---|---|
| 3 | $Mo_{12}P_{1,18}W_{1,8}Sb_{1,2}As_{0,1}Cu_{0,25}$ | 319 | 93,8 | 83 | 77,9 | 12 |
| 4 | $Mo_{12}P_{1,1}W_{0,9}Sb_{0,2}As_{0,2}Cu_{0,05}$ | 314 | 92,1 | 85 | 78,3 | 8 |
| 5 | $Mo_{12}P_{1,1}W_{0,9}Sb_{1}Cu_{0,25}$ | 284 | 91,7 | 84,6 | 77,5 | 6 |
| 6 | $Mo_{12}P_{1,1}W_{0,9}Sb_{1}As_{0,2}$ | 302 | 89,6 | 81,3 | 72,8 | 10 |
| 7 | $Mo_{12}P_{1,1}W_{0,9}Sb_{1}As_{0,2}Cu_{0,1}Mn_{0,1}$ | 318 | 91,7 | 84,5 | 77,4 | 10 |
| 8 | $Mo_{12}P_{1,1}W_{0,9}Sb_{1}As_{0,2}Cu_{0,1}Nb_{0,4}$ | 324 | 95 | 87,2 | 82,8 | 7 |
| 9 | $Mo_{12}P_{1,1}W_{0,9}Sb_{1}As_{0,2}Cu_{0,1}Fe_{0,1}$ | 312 | 94,4 | 85,9 | 81,1 | 11 |
| 10 | $Mo_{12}P_{1,1}W_{0,9}Sb_{1}As_{0,2}Cu_{0,1}Sn_{0,05}$ | 316 | 91,1 | 84,0 | 76,5 | 8 |
| 11 | $Mo_{12}P_{1,1}W_{0,9}Sb_{1}As_{0,2}Cu_{0,1}Cr_{0,05}$ | 316 | 92,9 | 85,2 | 79,2 | 10 |
| 12 | $Mo_{12}P_{1,2}W_{1,8}Sb_{1}As_{0,2}Cu_{0,25}K_{0,03}$ | 319 | 95,6 | 81,9 | 78,3 | 7 |
| 13 | $Mo_{12}P_{1,1}W_{0,9}Sb_{1}As_{0,2}Cu_{0,25}Cs_{0,09}$ | 326 | 92,2 | 81,9 | 75,6 | 4 |
| 14 | $Mo_{12}P_{1,1}W_{0,9}Sb_{1}As_{0,2}Cu_{0,25}Rb_{0,03}$ | 319 | 91,5 | 83,5 | 76,4 | 7 |

Tabelle 2

| Beispiel | Zusatz | Mole je Mol Mo | Badtemperatur [°C] | Umsatz [Mol-%] | Selektivität [Mol-%] | Ausbeute [Mol-%] |
|---|---|---|---|---|---|---|
| 15 | Weinsäure | 0,11 | 312 | 87,4 | 84,5 | 73,8 |
| 16 | Zitronensäure | 0,08 | 300 | 83,5 | 85,2 | 71,2 |
| 17 | Oxalsäure | 0,18 | 316 | 81 | 93,2 | 75,5 |

**Patentanspruch**

Verfahren zur Herstellung von Methacrylsäure durch Oxidation von Methacrolein mit Sauerstoff und Wasserdampf enthaltenden Gasgemischen bei Temperaturen von 200 bis 340°C aus Katalysatoren die Molybdän, Wolfram, Antimon und Phosphor enhalten, und Abtrennen der Methacrylsäure aus den Reaktionsgasen in an sich üblicher Weise, dadurch gekennzeichnet, dass man die Oxidation an einem Katalysator durchführt, der die allgemeine Formel

$Mo_{12}P_aW_bSb_cAs_dCu_eX_fY_gO_x$

hat in der
X für Nb, Fe, Mn, Sn und/oder Cr,
Y für ein Alkalimetall
a für 0,1 bis 3,
b für 0,1 bis 4,
c für > 0 bis 2,
d für 0 bis 1,
e für 0 bis 1,
d+e für > 0 bis 2,
f für 0 bis 1,5,
e+f für 0 bis 2,
g für 0 bis < 0,1 und
x für die zur Absättigung der Valenzen der Katalysatorkomponenten formal erforderliche Anzahl der Sauerstoffatome stehen.

**Claim**

A process for the preparation of methacrylic acid by oxidizing methacrolein by means of a gas mixture containing oxygen and steam, at from 200 to 340°C over a catalyst which contains molybdenum, tungsten, antimony and phosphorus, and isolating the methacrylic acid from the reaction gases in a conventional manner, wherein the oxidation is carried out over a catalyst of the general formula

$Mo_{12}P_aW_bSb_cAs_dCu_eX_fY_gO_x$

where
X is Nb, Fe, Mn, Sn and/or Cr,
Y is an alkali metal,
a is 0.1–3,
b is 0.1–4,
c is > 0–2,
d is 0–1,
e is 0–1,
d + e is > 0–2,
f is 0–1.5,
e + f is 0–2,
g is 0 to <0.1, and
x is the number of oxygen atoms formally required to saturate the valencies of the catalyst components.

**Revendication**

Procédé de préparation de l'acide méthacrylique par oxydation de la méthacroléine par des mélanges gazeux contenant de la vapeur d'eau et de l'oxygène, à des températures de 200 à 340°C en présence de catalyseurs au molybdène, au tungstène, à l'antimoine et au phosphore, suivie de la séparation de l'acide méthacrylique des mélanges réactionnels gazeux par une technique usuelle, caractérisé en ce que l'on réalise l'oxydation en présence d'un catalyseur de la formule générale

$Mo_{12}P_aW_bSb_cAs_dCu_eX_fY_gO_x$,

dans laquelle
X = Nb, Fe, Mn, Sn et(ou) Cr,
Y = un métal alcalin,
a vaut 0,1 à 3,
b vaut 0,1 à 4,
c vaut > 0 à 2,
d vaut 0 à 1,
e vaut 0 à 1 et d + e vaut > 0 à 2,
f vaut 0 à 1,5 et e + f vaut 0 à 2,
g vaut 0 à < 0,1 et,
x indique le nombre d'atomes d'oxygène en principe nécessaires pour la saturation des valences des composants du catalyseur.